# FASCICULE DE BREVET EUROPEEN

(11) **EP 1 676 559 B1**
(45) Date de publication et mention de la délivrance du brevet: **27.04.2016**
(21) Numéro de dépôt: 05301104.5
(22) Date de dépôt: 23.12.2005
(51) Int. Cl.: A61K 8/02, A61Q 5/12, A61Q 19/00, A61Q 1/02, A61Q 1/14, A61Q 9/02, A61Q 15/00, A61K 8/81, A61Q 19/10

(54) **Article cosmétique ou dermatologique comportant un support soluble dans l'eau**
Artikel für die Kosmetik oder die Dermatologie, der einen wasserlöslichen Träger enthält
Cosmetic or dermatological article containing a support which is soluble in water

(30) Priorité: 03.01.2005 FR 0550014
(43) Date de publication de la demande: 05.07.2006
(73) Titulaire: L'Oréal, 75008 Paris (FR)
(72) Inventeur: Simon, Pascal, 94320, THIAIS (FR)
(74) Mandataire: Tanty, François

(56) Documents cités:
- EP-A- 0 653 635
- EP-A- 1 454 950
- WO-A-00/30578
- WO-A-02/17853
- WO-A-96/25910
- WO-A-03/037282
- WO-A-2004/032859
- WO-A-2004/052347
- WO-A1-2005/023323
- GB-A- 2 302 651
- US-A- 3 803 300
- US-A- 5 567 431
- US-A- 5 629 003
- US-A1- 2003 099 692
- US-A1- 2004 137 041
- US-A1- 2004 247 649
- US-B1- 6 175 054
- DATABASE CA CHEMICAL ABSTRACTS SERVICE, COLUMBUS, OHIO, US; XP002358345 extrait de STN Database accession no. 107: 46 074 & JP 62 072609 A (SUNSTAR, INC.) 3 avril 1987 (1987-04-03)

## Description

La présente invention concerne des articles cosmétiques comportant au moins un support et au moins une composition de maquillage portée par le support.

Des articles comportant un support souple insoluble dans l'eau et une composition cosmétique portée par le support, encore appelés lingettes, sont très largement utilisés, pour le démaquillage notamment.

Des lingettes humides imprégnées d'une composition aqueuse moussante sont ainsi connues par les publications WO 02/092050, WO 02/092052 et US 6 287 757, entre autres,

Le brevet US 4 303 543 divulgue des lingettes sèches qui sont imprégnées d'une composition moussante durant la fabrication puis séchées après imprégnation.

La publication WO 00/07561 décrit des éponges bicouche renfermant une dose de poudre moussante.

Les lingettes ou éponges connues comportent un support constitué d'un substrat insoluble dans l'eau et sont une source de déchets solides.

Le brevet US 6 818 603 divulgue un savon en pain incorporant des fibres, lesquelles peuvent être hydrosolubles.

Le brevet US 6 576 575 décrit un pansement adhésif comportant des fibres solubles dans l'eau.

US 6 175 054 divulgue un film soluble permettant de délivrer un actif à une blessure.

WO 00/30578 divulgue un pansement portant une composition thérapeutique.

WO 2004/052347 divulgue un médicament.

US 5 629 003 divulgue des produits destinés à une application buccale ou à être ingérés. Il est par exemple mentionné que le produit se dissout complètement dans la bouche.

WO 02/17853 divulgue des composés utilisés comme antiadhésifs pouvant être mis en place par un chirurgien après une opération avant de refermer la plaie.

EP 0 653 635 divulgue un article portant une substance physiologiquement active pouvant être utilisé pour des examens cliniques.

US 5 567 431 divulgue un article destiné à être placé par un chirurgien sur le corps humain après une opération avant de refermer la plaie.

US 2004/0137041 divulgue un composé pour l'hygiène buccale.

WO 96/25910 divulgue des composés adhérant à des surfaces muqueuses comme la cavité buccale

US 2004/0247649 divulgue un article destiné à être dissous dans la bouche.

WO 03/037282 divulgue des produits de soin personnel.

US 2003/0099692 divulgue un composé se dissolvant dans la bouche ou dans une cavité corporelle, le composé ayant comme constituant principal de l'amidon.

US 3 803 300 divulgue un pansement pour l'application sur la peau.

EP 1 454 950 divulgue des articles alimentaires, cosmétiques ou pharmaceutiques se dissolvant dans l'eau.

XP 002358345 divulgue une préparation pour le bain se dissolvant dans l'eau.

WO 2004/032859 divulgue un produit de soin personnel se dissolvant dans l'eau.

GB 2 302 651 divulgue un patch à appliquer sur la peau et configuré pour procurer un effet rafraîchissant.

L'invention réside, selon l'un de ses aspects, dans un article cosmétique selon la revendication 1.

Les termes nappe et couche doivent être considérés comme synonymes.

Par « fluide », il faut comprendre « qui peut s'écouler sous l'effet de son propre poids ».

L'invention a encore pour objet, selon un autre de ses aspects, un ensemble comportant :
- un emballage,
- au moins un article tel que défini plus haut. Cet emballage peut comporter un témoin coloré représentatif de la couleur de la composition.

Le support peut notamment porter une composition cosmétique.

La composition cosmétique est par exemple contenue dans le support ou retenue sur celui-ci.

Par « soluble dans l'eau à une température inférieure à 20 °C », il faut comprendre une solubilisation dans l'eau à une température inférieure à 20 °C avec l'aide d'une agitation manuelle et/ou d'une friction du support le cas échéant, dans un laps de temps typiquement inférieur à 5 mn, de préférence inférieur à 1 mn, de préférence inférieur à 30 secondes. L'invention n'exclut pas qu'une eau de température supérieure à 20 °C soit utilisée pour dissoudre le support.

Par « souple », il faut comprendre un article pouvant être comprimé ou fléchir sans se rompre, capable de s'adapter aux reliefs du corps humain. Un article souple réalisé sous la forme d'une nappe fibreuse peut dans certains exemples de réalisation être replié sur lui-même au moins une fois sans se casser en deux morceaux.

L'article est par exemple à usage unique.

L'invention telle que définie ci-dessus présente l'avantage de réduire la quantité de déchets solides produits par le consommateur.

Un article réalisé conformément à l'invention peut offrir également un attrait supplémentaire, lié au changement d'aspect à l'utilisation, dû à la solubilisation du support.

L'invention offre encore de nouvelles possibilités pour le conditionnement et la formulation de nombreux produits cosmétiques tels que les produits de maquillage.

L'invention peut également permettre le conditionnement de compositions cosmétiques pulvérulentes se prêtant mal aux modes de conditionnement conventionnels, tels que le compactage dans une coupelle ou l'incorporation dans une pâte, par exemple.

L'invention peut aussi faciliter le conditionnement de compositions cosmétiques pâteuses ou pulvérulentes, difficiles à prélever manuellement.

La composition peut représenter entre 10 et 1000 % en poids, par rapport au poids du support.

### Support

Le support peut se présenter sous la forme d'un ou plusieurs films non fibreux, obtenus par exemple par évaporation d'un solvant, mais de préférence le support comporte des fibres enchevêtrées solubles dans l'eau, de préférence à une température inférieure à 20 °C.

Dans un exemple de mise en oeuvre de l'invention, le support comporte au moins une couche d'un non tissé, constituée essentiellement de fibres solubles dans l'eau, de préférence à une température inférieure à 20 °C, et peut comporter au moins une couche d'un substrat insoluble dans l'eau.

Le support peut être sensiblement non rétractable une fois mouillé.

Pour fabriquer le support dans le cas où celui-ci comporte un non-tissé, toutes les techniques appropriées de constitution d'un non-tissé à partir de fibres peuvent être utilisées.

Par exemple, les fibres peuvent être formées par extrusion et déposées sur un convoyeur pour former une nappe de fibres qui est ensuite consolidée par une technique classique de liage de fibres telle que par exemple l'aiguilletage, le liage à chaud, le calandrage ou le liage par jets d'air chaud (en anglais *air through bonding),* technique dans laquelle la nappe passe dans un tunnel où est insufflé de l'air chaud. Cette dernière technique est avantageusement utilisée lorsque la nappe est constituée de fibres bicomposant, par exemple des fibres comprenant au moins deux grades d'alcool polyvinylique (PVA), dont les points de fusion ou de ramollissement sont différents, ces fibres étant par exemple co-extrudées de manière à ce que la fibre soit constituée d'au moins un premier grade localisé au coeur de la fibre et d'au moins un deuxième grade localisé en périphérie de la fibre, sous la forme d'une gaine. Cela peut faciliter le liage des fibres lorsque la gaine présente un point de fusion plus faible que le coeur.

La nappe de fibres servant à la fabrication du non-tissé peut encore être formée par cardage de fibres découpées à une longueur de 10 à 50 mm, puis dépôt des fibres sur un convoyeur où la nappe peut ensuite être consolidée par une technique de liage telle que décrite ci-dessus.

La densité du support pourra dépendre des applications. Le support présente une densité inférieure ou égale à 0,1 g/cm³. Une densité inférieure ou égale à 0,1 g/cm³, mieux entre 0,01 g/cm³ et 0,1 g/cm³, peut le rendre très aéré et faciliter sa dissolution dans l'eau.

Dans un exemple de réalisation, le support comporte au moins deux couches de non tissé constituées chacune essentiellement de fibres solubles dans l'eau, de préférence à une température inférieure à 20 °C.

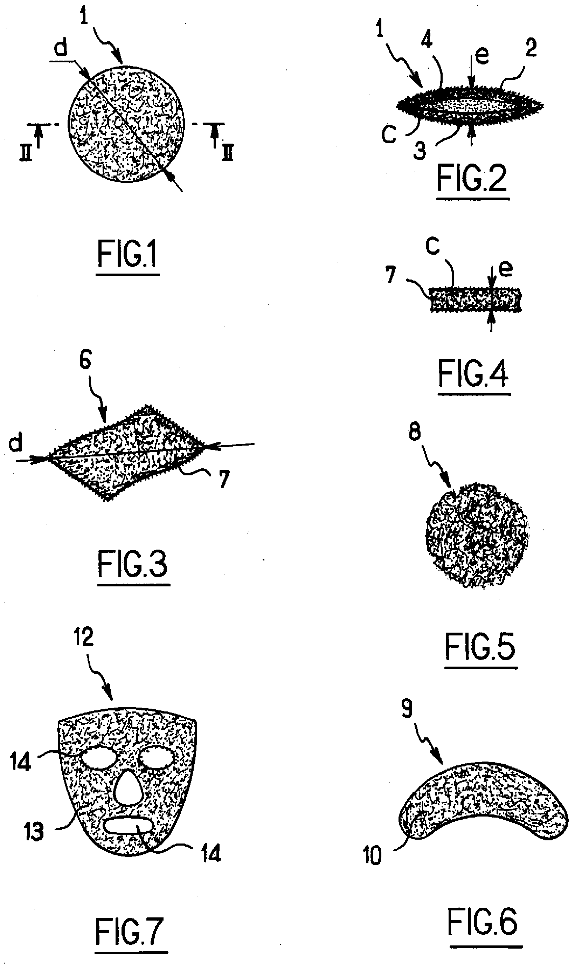

Les deux couches sont par exemple assemblées à leur périphérie, comme illustré sur les figures 1 et 2.

La figure 1 représente, en vue de dessus, un article 1 comportant deux feuilles 2 et 3 d'un non tissé formé de fibres hydrosolubles, assemblées par thermosoudage à leur périphérie de manière à constituer un coussinet capable de retenir dans une cavité intérieure 4 une composition C cosmétique comme on peut le voir sur la figure 2 (laquelle est une coupe transversale selon II-II de la figure 1).

Le support peut encore ne comporter qu'une seule couche de fibres hydrosolubles et une composition cosmétique peut par exemple être dispersée au sein de cette couche ou en recouvrir l'une des faces seulement.

On a représenté de manière schématique en perspective à la figure 3 un article cosmétique 6 qui comporte une couche unique 7 formée d'un non tissé de fibres solubles et dans laquelle la composition C est dispersée, celle-ci étant par exemple sous une forme pulvérulente, comme illustré à la figure 4 (laquelle est une coupe transversale partielle et schématique de la figure 3).

Sur la figure 3, l'article présente une forme rectangulaire mais l'article pourrait prendre une autre forme, par exemple ronde ou ovale, ayant par exemple des dimensions permettant sa préhension entre deux doigts au moins. L'article peut former une lingette.

Dans les exemples des figures 1 et 3, le support présente une forme générale aplatie, l'épaisseur maximale e sur les figures 2 et 4 étant par exemple inférieure à 10 mm pour une surface comprise entre 0,00005 m² et 0,01 m², de préférence comprise entre 0,0001 m² et 0,001m².

Le support peut encore présenter une forme générale non aplatie, présentant par exemple l'aspect d'un bloc, comme illustré à la figure 5, cette dernière représentant en vue de dessus un article 8 formé d'un amas globulaire de fibres hydrosolubles compactées, incorporant la composition cosmétique.

Dans un exemple de mise en oeuvre de l'invention, l'article formé par la composition cosmétique et le support est destiné à être mis en contact avec l'eau avant son utilisation. Le support est ainsi d'abord solubilisé entièrement avant que l'article ne soit appliqué sur le corps humain. Selon la quantité d'eau rajoutée à l'article pour solubiliser le support, on peut aisément ajuster la viscosité apparente de la composition obtenue.

Dans une variante de mise en oeuvre de l'invention, l'article formé par la composition cosmétique et le support est destiné à être mis en contact avec de l'eau pour son utilisation, mais le support est mis au contact d'une région du corps humain, par exemple la peau avant sa solubilisation complète, voire avant d'être mouillé. Cela peut permettre par exemple, selon la quantité d'eau ajoutée, de modifier les propriétés en fonction du résultat souhaité. L'eau peut être versée sur l'article alors que celui-ci n'est pas au contact du corps. Ce dernier peut encore être mouillé ou de l'eau être projetée ou versée sur le support alors que l'article est au contact de la région à traiter.

Dans un autre exemple encore de mise en oeuvre de l'invention, l'article est prévu pour être utilisé sans être imprégné par de l'eau. Dans ce cas, le support peut n'être dissout qu'après l'utilisation, si l'utilisateur le souhaite, en étant par exemple déposé au fond d'un lavabo et en faisant couler de l'eau dessus. L'article est par exemple préimprégné par la composition ou l'utilisateur peut déposer la composition dessus.

Notamment lorsque le support n'est pas destiné à être dissout entièrement dans l'eau préalablement à l'application, le support peut présenter une forme qui dépend de la région du corps à traiter.

On a ainsi représenté, en vue de dessus à la figure 6, un article 9 dont le support 10 est de contour réniforme, étant destiné par exemple au maquillage des paupières.

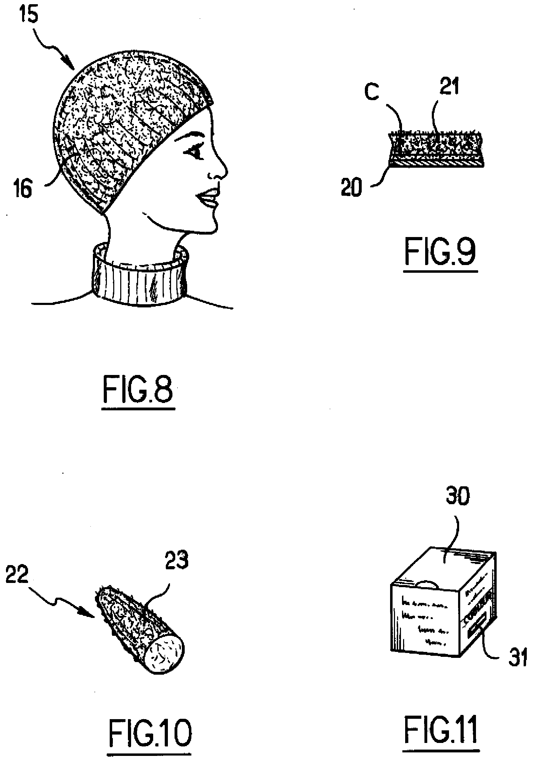

On a représenté en vue de face à la figure 7 un article 12 dont le support 13 forme un masque, avec des ouvertures ou découpes 14 pour les yeux, le nez et la bouche, et sur la figure 8 on a représenté de manière schématique en perspective un article 15 dont le support 16 forme une charlotte.

### Articles cosmétiques comportant un support non entièrement soluble dans l'eau, notamment dans l'eau à moins de 20 °C

L'invention réside encore, selon un autre de ses aspects, dans un article cosmétique, de préférence souple, comportant :
- au moins un support présentant une structure multicouche avec
   - au moins une couche entièrement soluble dans l'eau, notamment à une température inférieure à 20 °C,
   - au moins une couche d'un substrat insoluble dans l'eau, notamment à une température inférieure à 20 °C,
- au moins une composition de maquillage portée par le support.

Un tel support peut par exemple comporter, comme illustré à la figure 9 (qui représente de manière schématique en coupe un article selon cet aspect de l'invention), une couche 20 d'un substrat insoluble dans l'eau recouverte par une couche 21 constituée de fibres hydrosolubles, la composition cosmétique C étant par exemple retenue entre les couches 20 et 21, comme illustré. Les couches 20 et 21 sont par exemple assemblées à leur périphérie par thermosoudage ou autrement.

Le substrat 20 est par exemple une feuille d'un non-tissé synthétique, par exemple un non-tissé de fibres de polyéthylène, de polypropylène, de polyéthylène téréphtalate (PET), d'acide polylactique, de polyamide, de viscose, de cellulose ou d'un mélange de ces fibres ... ou un film, pouvant être perméable ou non. Les non tissés sont décrits de façon générale dans RIEDEL « Nonwoven Bonding Methods & Materials », Nonwoven World (1987).

Une structure multicouche avec au moins une couche formée d'un substrat insoluble dans l'eau peut par exemple être utile pour réaliser un article 22 comportant un support 23 en forme de doigt de gant, comme illustré à la figure 10 (qui est une perspective schématique). La couche formée de fibres hydrosolubles est située à l'extérieur de l'article, étant destinée à se solubiliser lors de l'utilisation, après avoir été mouillée ou en venant au contact d'une région mouillée du corps.

La couche 20 de substrat insoluble peut encore présenter la forme d'un gant ou d'une moufle.

Lorsque le support comporte plusieurs couches, que celles-ci soient toutes réalisées avec des fibres hydrosolubles ou non, les différentes couches peuvent être assemblées de multiples manières, par exemple par soudage, collage ou couture, et ces couches peuvent constituer le cas échéant une ou plusieurs cavités contenant une ou plusieurs compositions cosmétiques ou plusieurs composants d'une même composition cosmétique à mélanger extemporanément. Lors d'un assemblage par couture, un fil lui-même hydrosoluble peut être utilisé, le cas échéant.

### Fibres hydrosolubles

Comme indiqué plus haut, le support peut comporter, voire être constitué essentiellement, par des fibres solubles, de préférence dans l'eau à moins de 20 °C.

Le support peut ainsi comporter, par exemple, plus de 95 % en masse, voire plus de 99 % ou davantage encore de fibres hydrosolubles.

De préférence, les fibres sont réalisées avec de l'alcool polyvinylique (PVA) selon un procédé qui leur confère la solubilité recherchée.

Des fibres solubles dans l'eau à une température inférieure à 20 °C sont commercialisées par la société japonaise KURARAY sous la dénomination commerciale KURALON K-II WN2. Le procédé de fabrication de ces fibres comporte l'emploi de solvants organiques. La section de ces fibres peut être sensiblement circulaire.

La demande EP 0 636 716, décrit des fibres hydrosolubles à base de PVA et leur procédé de fabrication.

L'invention n'est pas limitée à l'emploi de PVA et des fibres réalisées dans d'autres matériaux hydrosolubles peuvent être utilisées sous réserve de se dissoudre dans de l'eau ayant la température recherchée, par exemple des fibresde polysaccharides commercialisées sous la dénomination LYSORB par la société LYSAC TECHNOLOGIES, INC ou des fibres à base de polymères polyholosides comme le glucomannane ou l'amidon.

Le support peut comporter, le cas échéant, un mélange de fibres solubles dans l'eau à des températures différentes.

Les fibres peuvent être composites, et comporter par exemple un coeur et une gaine n'ayant pas la même nature, par exemple formés de différents grades de PVA.

Le support peut être sensiblement dépourvu de fibres insolubles dans l'eau.

Selon un aspect de l'invention, le support est dépourvu d'adhésif, notamment d'adhésif sensible à la pression.

### Composés cosmétiques

Des composés très divers peuvent être utilisés dans un article réalisé conformément à l'invention.

Il s'agit de composés destinés au maquillage.

La composition cosmétique portée par le support peut représenter par exemple entre 10 et 1000 % en poids, par rapport au poids du support.

### Actifs

L'article comporte au moins un actif cosmétique

Ces actifs peuvent être utilisés par exemple à des concentrations de 0 à 20 % et notamment de 0,001 à 15 %, par rapport au poids total de la composition.

Toute ou partie des actifs peut être encapsulée, le cas échéant.

### Agents de coloration

L'article comporte au moins un agent de coloration, en quantité comprise entre 0 et 100%, par exemple entre 0 à 95 %, de préférence entre 0,1 à 50 %, et mieux de 1 à 25 % du poids total de la composition, notamment au moins un pigment ou colorant, par exemple un agent de coloration choisi parmi les pigments minéraux, les pigments ou laques organiques, les pigments nacrés, les pigments composites, les colorants liposolubles et hydrosolubles.

Les pigments minéraux peuvent être enrobés ou non. On peut citer le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique.

Les pigments nacrés peuvent être choisis parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, les nacres de couleur or telles que commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges. Les colorants liposolubles sont par exemple des extraits végétaux, le rouge Soudan, le DC Red 17, le DC Green 6, le β-carotène, l'huile de soja, le brun Soudan, le DC Yellow 11, le DC Violet 2, le DC orange 5, le jaune quinoléine.

Les colorants hydrosolubles sont choisis par exemple parmi les extraits végétaux, notamment le jus de betterave et le bleu de méthylène.

L'agent de coloration peut comporter au moins une matière colorante organique, par exemple au moins un pigment organique et/ou au moins une laqueorganique.

La matière colorante organique peut comporter par exemple des pigments ou laques organiques qui peuvent être choisis parmi les composés ci-dessous et leurs mélanges :
- le carmin de cochenille,
- les pigments organiques de colorants azoïques, anthraquinoniques, indigoïdes, xanthéniques, pyréniques, quinoliniques, de triphénylméthane, de fluorane,
- les laques organiques ou sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.

Parmi les pigments organiques, on peut notamment citer ceux connus sous les dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

La matière colorante organique peut comporter une laque organique supportée par un support organique tel que la colophane ou le benzoate d'aluminium, par exemple.

Parmi les laques organiques, on peut en particulier citer celles connues sous les dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

Les composés correspondant à chacune des matières colorantes organiques citées précédemment sont mentionnés dans l'ouvrage « International Cosmetic Ingredient Dictionnary and Handbook », Edition 1997, pages 371 à 386 et 524 à 528, publié par « The Cosmetic, Toiletry, and Fragrance Association ».

L'article peut comporter au moins un agent de coloration goniochromatique.

L'agent de coloration goniochromatique peut être choisi par exemple parmi les structures multicouche interférentielles et les agents de coloration à cristaux liquides, par exemple Fc₂O₃/SiO₂/Fe₂O₃/SiO₂/Fe₂O₃, un pigment ayant cette structure étant commercialisé sous la dénomination SICOPEARL par la société BASF, MoS₂/SiO₂/mica-oxyde/SiO₂/MoS₂, Fe₂O₃/SiO₂/mica-oxyde/SiO₂/Fe₂O₃, TiO₂/SiO₂/TiO₂ ou TiO₂/Al₂O₃/TiO₂, des pigments ayant ces structures étant commercialisés sous la dénomination XIRONA par la société MERCK (Darmstadt).

L'agent de coloration peut encore comporter un pigment diffractant, présentant par exemple la structure MgF₂/Al/MgF₂, tel que commercialisé sous la dénomination SPECTRAFLAIR 1400 Pigment Silver par la société FLEX PRODUCTS, ou SPECTRAFLAIR 1400 Pigment Silver FG.

Quelle que soit la nature des pigments utilisés, l'article peut comporter outre la phase particulaire comportant les pigments au moins un liant comprenant au moins un organopolysiloxane hydrophile solide élastomérique. Des exemples de tels polymères sont donnés dans la demande US 2004/0071648.

### Agents filmogènes

L'article peut comporter un ou plusieurs agents filmogènes, par exemple lorsqu'il est destiné au maquillage ou à former un film pelable ou un masque de soin.

Le support lui-même peut servir d'agent filmogène après dissolution, notamment lorsque le support est réalisé en PVA.

Le support peut ainsi être en une quantité suffisante pour permettre la formation d'un film continu sur des matières kératiniques.

L'article peut comporter d'autres agents filmogènes, lesquels pourront être choisis en fonction par exemple de la nature du support.

Ces autres agents filmogènes peuvent être choisis parmi les polymères synthétiques, de type radicalaire ou de type polycondensat, les polymères d'origine naturelle, et leurs mélanges.

Les polymères filmogènes de type radicalaire peuvent être par exemple des polymères ou des copolymères vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides comme les acides carboxyliques insaturés α,β-éthyléniques.

Parmi les polycondensats filmogènes, on peut citer les polyuréthanes, les polyesters, les polyesters amides, les polyamides, et les polyurées.

Les polymères d'origine naturelle, éventuellement modifiés, peuvent être choisis parmi la résine shellac, la gomme de sandaraque, les dammars, les élémis, les copals, les polymères cellulosiques.

### Autres ingrédients

L'article peut également contenir tous ingrédients couramment utilisés en cosmétique et physiologiquement acceptables, tels que par exemple des charges, épaississants, des tensioactifs, des oligo-éléments, des séquestrants, des parfums, des agents alcalinisants ou acidifiants, des conservateurs et des antioxydants.

L'article peut comporter par exemple au moins un composé destiné à permettre la formation de mousse lorsque l'article est mis au contact d'eau en présence d'une agitation, par exemple au moins un tensio-actif anionique, cationique ou amphotère ou non ionique. Comme tensioactifs de ce type, on peut citer par exemple :
(1) parmi les tensioactifs non ioniques, les polymères blocs oxyéthylénés oxypropylénés tels que le Poloxamer 184 (nom CTFA) ; les alkylpolyglycosides et notamment les alkylpolyglucosides (APG) ayant un groupe alkyle comportant de 6 à 30 atomes de carbone (alkyl-C₆-C₃₀ polyglucosides) et de préférence 8 à 16 atomes de carbone, comme par exemple le decylglucoside (Alkyl-C9/C11-polyglucoside (1.4) tel que le produit commercialisé sous la dénomination MYDOL 10 par la société Kao Chemicals, le produit commercialisé sous la dénomination PLANTAREN 2000 UP ou PLANTACARE 2000 UP par la société Henkel, et le produit commercialisé sous la dénomination ORAMIX NS 10 par la société Seppic ; le caprylyl/capryl glucoside comme le produit commercialisé sous la dénomination ORAMIX CG 110 par la Société Seppic ; le laurylglucoside comme les produits commercialisés sous les dénominations PLANTAREN 1200 N et PLANTACARE 1200 par la société Henkel ; et le coco-glucoside comme le produit commercialisé sous la dénomination PLANTACARE 818/UP par la société Henkel ;
(2) parmi les tensioactifs anioniques, les alkylsulfates, les alkyl éther sulfates et leurs sels, notamment leurs sels de sodium, comme le mélange de Sodium Laureth Sulfate / Magnésium Laureth Sulfate / Sodium Laureth-8 Sulfate / Magnésium Laureth-8 Sulfate, vendu sous le nom de Texapon ASV par la société Henkel ; le lauryl éther sulfate de sodium (C12-14 70/30) (2,2 OE) commercialisé sous les dénominations SIPON AOS 225 ou TEXAPON N702 PATE par la société Henkel, le lauryl éther sulfate d'ammonium (C12-14 70/30) (3 OE) commercialisé sous la dénomination SIPON LEA 370 par la société Henkel ; l'alkyl (C12-C14) éther (9 OE) sulfate d'ammonium commercialisé sous la dénomination RHODAPEX AB/20 par la société Rhodia Chimie ;
(3) parmi les tensioactifs amphotères ou zwitterioniques, les dérivés alkylamido alkylamines tels que le N-cocoyl-N-carboxyméthoxyéthyl-N-carboxyméthyl-éthylènediamine N-di-sodique (nom CTFA : Disodium cocoampho-diacetate) commercialisé en solution aqueuse saline sous la dénomination MIRANOL C2M CONC NP par la société Rhodia Chimie ; le N-cocoyl-N-hydroxyéthyl-N-carboxyméthyl-éthylènediamine N-sodique (nom CTFA : sodium cocampho-acetate) et le mélange d'éthanolamides d'acide de coco (nom CTFA : Cocamide DEA).

L'article peut comprendre aussi un mélange de ces tensioactifs.

L'article peut comporter des composés destinés à réagir ensemble en présence d'eau, par exemple pour provoquer une effervescence, par exemple au moins un acide organique, notamment l'acide citrique, et au moins un agent alcalin, par exemple le bicarbonate de soude

### Formes d'incorporation du ou des composés cosmétiques dans l'article

Au moins un composé cosmétique et plus généralement la composition cosmétique peut être présente ou non à l'extérieur de l'article.

Lorsque la composition est présente à l'extérieur de l'article, ce dernier peut être utilisé par exemple en étant amené au contact de la région à traiter, de manière à permettre à la composition de transférer. Ce transfert peut s'effectuer, le cas échéant, en frottant l'article sur la région à traiter.

Dans le cas où la composition est présente à l'extérieur de l'article, ce dernier peut ne pas être mouillé lors de l'utilisation, la solubilisation du support n'ayant lieu qu'après l'utilisation.

Lorsque la composition est présente à l'extérieur du support, l'article peut néanmoins dans certains cas être mouillé lors de l'utilisation, par exemple pour modifier des propriétés de la composition, améliorer le transfert de la composition ou former un produit filmogène.

Lorsque la composition n'est pas présente à l'extérieur de l'article, le support peut avoir à être solubilisé au moins partiellement, voire entièrement, avant l'utilisation.

La composition peut être présente dans l'article au moins partiellement, voire totalement, sous une forme pulvérulente, notamment une forme pulvérulente sensiblement anhydre. L'article peut, par exemple, être sec au toucher. La granulométrie de la composition pourra être choisie en fonction de la porosité du support de façon à diminuer les pertes au secouage.

La composition, lorsqu'elle est pulvérulente, peut être contenue dans une cavité du support, par exemple formée entre deux couches de celui-ci comme c'est le cas de l'exemple de réalisation de la figure 2, ou être dispersée, par exemple de manière homogène, au sein du support, notamment au sein d'une couche fibreuse unique du support comme c'est le cas de l'exemple de réalisation de la figure 4.

La composition peut ne pas être pulvérulente mais être liquide, crémeuse ou pâteuse, formant par exemple une pâte. Dans ce cas, la composition comporte par exemple un ou plusieurs solvants compatibles avec la nature du support, notamment des solvants non aqueux, de façon à éviter sa dissolution prématurée, comme par exemple les alcools inférieurs comportant de 1 à 6 atomes de carbone, tels que l'éthanol ; les polyols tels que la glycérine ; les glycols comme le butylène glycol, l'isoprène glycol, l'hexylène glycol, le propylène glycol ; les polyéthylène glycols tels que le PEG-8, le sorbitol ; les huiles hydrocarbonées d'origine animale, telles que le perhydrosqualène ; les huiles hydrocarbonées d'origine végétale, telles que les glycérides mono- ou di- ou tri- esters d'acides gras et de glycérol, par exemple les huiles d'amande douce, de tournesol, de maïs, de soja, de noisette, d'abricot ; les triglycérides d'origine synthétique comme ceux vendus par la société Stearineries Dubois ou ceux vendus sous les dénominations Miglyol 810, 812 et 818 par la société Dynamit Nobel; les esters et les éthers de synthèse, notamment d'acides gras, comme les huiles de formules R¹ COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le stéarate d'octyl-2-dodécyle, l'isostéaryl lactate, l'octylhydroxystéarate, le citrate de triisocétyle ; les esters de polyol, comme le dioctanoate de propylène glycol, le diisononanoate de diéthylèneglycol ; et les esters du pentaérythritol ; les hydrocarbures linéaires ou ramifiés, d'origine minérale ou synthétique, tels que les huiles de paraffine, volatiles ou non ; les alcools gras ayant de 8 à 26 atomes de carbone, comme l'alcool cétylique, l'alcool stéarylique et leur mélange (alcool cétéarylique), l'octyldodécanol, le 2-hexyldécanol ; les alcools gras alcoxylés et notamment éthoxylés tels que l'oleth-12 ou le ceteareth-20 ; les huiles fluorées partiellement hydrocarbonées et/ou siliconées comme celles décrites dans le document JP-A-2-295912. Comme huiles fluorées, on peut citer aussi le perfluorométhylcyclopentane et le perfluoro-1,3 diméthylcyclohexane, vendus sous les dénominations de "FLUTEC PC1^{®}" et "FLUTEC PC3^{®}" par la Société BNFL Fluorochemicals ; les perfluoroalcanes tels que le dodécafluoropentane et le tétradécafluorohexane, vendus sous les dénominations de "PF 5050^{®}" et "PF 5060^{®}" par la Société 3M ; les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényldiméthicones, les diphénylméthyldiphényl trisiloxanes, les 2-phényléthyltriméthylsiloxysilicates, et les polyméthylphénylsiloxanes.

On entend par "huile hydrocarbonée" dans la liste des huiles citées ci-dessus, toute huile comportant majoritairement des atomes de carbone et d'hydrogène, et éventuellement des groupements ester, éther, fluoré, acide carboxylique et/ou alcool.

La composition pourra contenir une certaine quantité d'eau au moment de son imprégnation sur le support. De manière à éviter sa solubilisation prématurée, l'eau introduite sur le support lors de son imprégnation sera éliminée par les moyens classiquement utilisés pour la déshydratation des compositions contenant de l'eau, comme par exemple le chauffage.

La composition peut être déposée sur une face seulement du support, l'autre face du support pouvant être utilisée par exemple pour la préhension de l'article.

Dans le cas d'une composition colorée, l'article est conditionné par exemple dans un emballage tel qu'une boîte 30, comme illustré à la figure 11. Un témoin 31 indicatif de la couleur du produit obtenu après dissolution du support peut figurer sur l'emballage, le cas échéant, pour renseigner le consommateur avant l'achat.

Lorsque la composition est incorporée durant la fabrication de l'article dans celui-ci, l'article est par exemple conditionné en vrac dans une boîte ou dans un emballage individuel. Le cas échéant, les articles sont conditionnés en chapelet. Les articles peuvent encore être repliés sur eux-mêmes et intercalés, de telle sorte que le retrait d'un article amène le suivant dans une configuration facilitant sa préhension.

Lorsque la composition doit être déposée sur le support par l'utilisateur lui-même, la composition et le support peuvent être proposés sous la forme d'un kit, par exemple. La composition est par exemple livrée en une quantité suffisante pour permettre d'en distribuer une pluralité de doses sur un ensemble de supports destinés à être utilisés successivement.

### Exemples

### Applications au maquillage / Compositions transférables par simple contact ou friction sur la peau, les muqueuses ou les cheveux

Exemple A : Maquillage de la peau, notamment des paupières On réalise un support en découpant une forme ovoïde d'environ 5 cm de long et 1,5 cm de large à partir d'une nappe de KURALON K-II WN2 d'épaisseur environ 0,5mm et de grammage 60 g/m².

Selon l'intensité du maquillage recherché ce support est imprégné par une quantité comprise entre 0,02 g et 0,1 g de la composition pulvérulente colorée suivante, déposée sur une face de celui-ci.

| | |
|---|---|
| Mica | 14 % |
| Ferric Ferrocyanide | 6 % |
| Talc | 8 % |
| Calcium Sodium borosilicate | 60 % |
| Dioxyde de titane | 10 % |
| Ethylhexyl palmitate | 1,5 % |
| Conservateurs | 0,5 % |

Le support ainsi imprégné est doux au toucher et de préhension facile notamment par sa face opposée à celle portant la composition et peut être déplacé sur la peau de manière à permettre à la composition de transférer sur celle-ci.

Après utilisation, le support est facilement éliminé sous l'eau courante.

L'imprégnation du support peut s'effectuer au moment de l'utilisation ou le support peut être proposé à l'utilisateur pré-imprégné, dans un emballage individuel notamment.

### Exemple B : Maquillage de la peau, notamment des joues

On réalise un support en découpant une forme ronde d'environ 5 cm de diamètre dans une nappe de KURALON K-II WN2 d'épaisseur environ 2mm et de grammage 80 g/m².

Ce support est imprégné de 0,1g de la composition pulvérulente suivante, déposée sur une face de celui-ci.

| | |
|---|---|
| Mica | 30 % |
| Pigments d'oxydes de fer et d'oxydes de zinc | 3,5 % |
| Talc | 35 % |
| Dioxyde de titane | 5 % |
| Nylon 12 | 20 % |
| Ethylhexyl palmitate | 2 % |
| Huile minérale | 4 % |
| Conservateurs | 0,5 % |

Après utilisation, le support est facilement éliminé sous l'eau courante.

### Exemple C : Maquillage de la peau, notamment du visage

Le même support qu'à l'exemple A est enduit avec 0,25g de la composition de blush pâteuse anhydre suivante :

| | |
|---|---|
| Mica | 2 % |
| Pigments à base d'oxydes de fer | 3 % |
| Talc | 4 % |
| Nylon 12 | 2 % |
| Hydrogenated styrene isoprene copolymer | 10 % |
| Isohexadecane | 25 % |
| Disteardimonium hectorite | 2 % |
| Silice | 5 % |
| Isononyl isononanoate | 15 % |
| Polymethyl methacrylate | 15 % |
| Distillats de Petroleum | 17 % |

Après utilisation, le support est facilement éliminé par dissolution dans l'eau froide.

### Applications au maquillage / Articles dont le support est dissout avant l'application

### Exemple D : Maquillage de la peau

Un article tel que celui illustré aux figures 1 et 2 est réalisé avec des fibres KURALON K-II WN2 en thermosoudant à leur périphérie les couches 2 et 3, et en ayant préalablement introduit la composition cosmétique dans la cavité 4. Les couches 2 et 3 ont un grammage compris entre 70 et 80 g/m² et une épaisseur comprise entre 3 et 4 mm. L'article se présente sous la forme d'un disque de 3cm de diamètre contenant environ 0,3g d'une composition colorée pulvérulente ayant la formulation suivante :

| | |
|---|---|
| Oxyde de titane (anatase non traitée) | 20 % |
| Oxyde de fer jaune | 4,5 % |
| Oxyde de fer brun jaune | 4 % |
| Oxyde de fer noir | 1 % |
| Poudre de nylon (Orgasol de chez Atochem) | 30 % |
| Dimethicone/vinyl dimethicone crosspolymer | 35,5 % |
| glycerol | 4,5 % |
| conservateurs | 0,5 % |

Pour utiliser l'article, on dispose celui-ci dans le creux de la main et l'on verse de l'eau sur le support.

Les fibres de PVA contribuent à texturer la composition et/ou lui confèrent des propriétés filmogènes. L'utilisateur peut agir sur la texture du produit en ajoutant plus ou moins d'eau.

Le produit ainsi formé peut être étalé sur le corps ou le visage directement avec les doigts ou en utilisant un applicateur, par exemple un embout floqué, comme une mousse, un feutre, du coton ou un pinceau.

### Applications à l'hygiène et/ou au soin

### Exemple E : Hygiène des aisselles (exemple de référence)

On réalise un article comportant un support constitué par un disque de 7 cm de diamètre d'une nappe de fibres de KURALON K-II WN2 de grammage 80 g/m² et de 2 mm d'épaisseur, imprégné de 0,2 g d'une composition déodorante pulvérulente ayant la formulation suivante :

| | |
|---|---|
| Alcool cétylstéarylique | 2 % |
| Parfum | 0,5 % |
| Conservateurs | 0,5 % |
| Poly diméthyl siloxane | 0,5 % |
| Hydroxy chlorure d'aluminium | 96,5 % |

L'article s'utilise à sec, en essuyant les aisselles.

L'affinité pour l'eau du support permet d'essuyer d'éventuels résidus de transpiration.

### Exemple F : Nettoyage de la peau (exemple de référence)

Un article tel que celui illustré aux figures 1 et 2 est réalisé avec des fibres KURALON K-II WN2 en thermosoudant à leur périphérie les couches 2 et 3, et en ayant préalablement introduit la composition cosmétique dans la cavité 4. L'article se présente sous la forme d'un disque de 3cm de diamètre L'article contient environ 0,3g d'une composition de nettoyage pulvérulente ayant la formulation suivante :

| | |
|---|---|
| Amidon (octenyl succinyl starch) | 40 % |
| Sodium Cocoyl isethionate | 34,9 % |
| Potassium laurate | 10 % |
| Potassium myristate | 10 % |
| Parfum | 3 % |
| Acide salicylique | 2 % |
| Conservateurs | 0,1 % |

Pour utiliser l'article, on dispose celui-ci dans le creux de la main et l'on verse à peu près 3cm³ d'eau dessus.

On remue celui-ci avec l'index de l'autre main en rajoutant jusqu'à 3 à 6 cm³ d'eau si nécessaire, de manière à obtenir une mousse homogène après solubilisation complète du support.

Cette mousse peut être utilisée pour se laver les mains ou le visage, par exemple.

### Exemple G : Nettoyage de la peau (exemple de référence)

On réalise une lingette de la manière suivante.

On découpe une nappe de 10 cm de côté de grammage 60 g/m² et d'épaisseur 1 mm en KURALON K-II WN2 que l'on imprègne de 0,9 g de la composition moussante concentrée suivante :

| | |
|---|---|
| Sodium laureth sulfate à 70% dans l'eau (Texapon N702 de chez Cognis) | 50 % |
| Disodium cocoamphodiacétate à 39% dans l'eau saumurée (11% chlorure de sodium) Miranol C2M de chez Rhodia | 24,9 % |
| Lauroyl sarcosinate de sodium à 90% dans l'eau (Sarkosyl NL97 de chez Ciba Geigy) | 16,9 % |
| Parfum | 3 % |
| Glycérol | 5 % |
| Conservateurs | 0,3 % |

Cette composition contient au moins 15 % d'eau. Elle se présente sous la forme d'une pâte que l'on dépose sur le support par les moyens classiques, comme l'enduction par exemple.

On fait sécher la composition, et celle-ci adhère aux fibres une fois sèche.

Une fois sec, le support est calandré entre des rouleaux, pour le densifier, le rigidifier et diminuer la porosité en surface.

Pour utiliser l'article, la lingette est passée sous l'eau puis frictionnée pour créer la mousse et solubiliser le support.

### Exemple H : Rasage de la peau (exemple de référence)

On réalise un article destiné au rasage avec le même support que c l'exempleE, la composition de l'exemple précédent étant remplacée par la suivante :

| | |
|---|---|
| Acide stéarique | 10 % |
| Acide palmitique | 10 % |
| Acide myristique | 10 % |
| Dimethiconol stearate | 0,5 % |
| Disodium cocoamphodiacetate | 5 % |
| Potassium hydroxyde | 8 % |
| Eau | 51,2 % |
| Glycerol | 5 % |
| Conservateurs | 0,3 % |

Cette composition contient au moins 15 % d'eau. Elle se présente sous la forme d'une pâte que l'on dépose sur le support par les moyens classiques comme l'enduction par exemple.

On fait sécher la composition, et celle-ci adhère aux fibres une fois sèche.

Une fois sec, le support est calandré entre des rouleaux, pour le densifier, le rigidifier et diminuer la porosité en surface.

Pour utiliser l'article, celui-ci est appliqué sur les joues préalablement humectées, puis on frictionne en rajoutant graduellement de l'eau.

### Exemple I : Démaquillage (exemple de référence)

On réalise une compresse démaquillante destinée au démaquillage du visage avec le même support que celui de l'exemple E, la composition de l'exemple précédent étant remplacée par la suivante :

| | |
|---|---|
| Huile de Parleam | 40 % |
| Isohexadécane | 28 % |
| Palmitate de dextrine (Rheopearl TL) | 2 % |
| PEG-20 glyceryl tri-isostearate | 25 % |
| Simulgel 600 (nom CTFA : Acrylamide / Sodium acryloyldimethyltaurate copolymer / Isohexadecane / Polysorbate 80) | 5 % |

La composition est anhydre, elle est imprégnée sur le support par des moyens classiques comme par exemple des rouleaux lécheurs ou des rampes d'imprégnation.

Une émulsion démaquillante est générée en passant la feuille sur le visage mouillé ou mieux en la frottant dans le creux de la main avec un peu d'eau.

Bien entendu, l'invention n'est pas limitée aux exemples qui viennent d'être décrits.

On peut notamment utiliser d'autres actifs que ceux mentionnés ci-dessus.

Dans toute la description, y compris les revendications, les proportions sont massiques, sauf si le contraire est spécifié.

L'expression « comportant un » doit se comprendre comme étant synonyme de « comportant au moins un », sauf si le contraire est spécifié.

L'expression « compris entre » doit se comprendre bornes incluses.

## Revendications

1. Article cosmétique comportant :
- un support sous forme d'au moins une nappe d'un matériau soluble dans l'eau à une température inférieure à 20 °C, et présentant une densité inférieure ou égale à 0, 1 g/cm³, et
- au moins une composition de maquillage portée par le support,
l'article comportant un agent de coloration choisi parmi : les pigments minéraux, les pigments ou laques organiques, les pigments nacrés, les pigments composites, les colorants liposolubles et hydrosolubles, les pigments minéraux étant choisis parmi : le dioxyde de titane traité en surface, les oxydes de zirconium ou de cérium, ainsi que les oxydes de fer ou de chrome, le violet de manganèse, le bleu outremer, l'hydrate de chrome et le bleu ferrique.

2. Article selon la revendication 1, **caractérisé par le fait qu'**il est souple.

3. Article selon la revendication 1, **caractérisé par le fait qu'**il est non adhésif.

4. Article selon l'une quelconque des revendications 1 à 3, **caractérisé par le fait que** le support est entièrement soluble dans l'eau.

5. Article selon l'une quelconque des revendications 1 à 4, dans lequel le support comporte des fibres solubles dans l'eau.

6. Article selon la revendication 5, dans lequel le support est constitué essentiellement par lesdites fibres solubles.

7. Article selon l'une quelconque des revendications 1 à 6, dans lequel le support comporte au moins une couche d'un non tissé constituée essentiellement de fibres solubles dans l'eau.

8. Article selon la revendication précédente, **caractérisé par le fait que** le support comporte au moins deux couches de non tissé constituées chacune essentiellement de fibres solubles dans l'eau.

9. Article selon la revendication précédente, **caractérisé par le fait que** les deux couches sont formées par deux feuilles assemblées à leur périphérie.

10. Article selon la revendication précédente, **caractérisé par le fait que** les feuilles sont thermosoudées.

11. Article selon la revendication 2, **caractérisé par le fait que** la composition de maquillage est dispersée au moins partiellement au sein du support.

12. Article selon la revendication 8, **caractérisé par le fait que** la composition de maquillage est disposée au moins partiellement entre les couches, mieux est disposé sensiblement entièrement entre les couches.

13. Article selon l'une quelconque des revendications précédentes, présentant une forme générale aplatie.

14. Article selon l'une quelconque des revendications 1 à 12, formant un bloc.

15. Article selon la revendication 1, **caractérisé par le fait qu'**il comporte au moins une couche constituée essentiellement de fibres solubles dans l'eau et au moins une couche d'un substrat insoluble dans l'eau.

16. Article selon la revendication 5 ou 6, dans lequel les fibres comportent de l'alcool polyvinylique.

17. Article selon la revendication 16, dans lequel les fibres présentent une section transversale pleine sensiblement circulaire.

18. Article selon l'une quelconque des revendications précédentes, **caractérisé par le fait que** la composition de maquillage est une composition pulvérulente.

19. Article selon l'une quelconque des revendications 1 à 17, **caractérisé par le fait que** la composition de maquillage est une composition pâteuse.

20. Article selon l'une quelconque des revendications 1 à 17, **caractérisé par le fait que** la composition de maquillage est une composition moussante.

21. Article selon l'une quelconque des revendications 1 à 20, **caractérisé par le fait qu'**il comporte une composition de maquillage de la peau, des muqueuses ou des phanères.

22. Article selon l'une quelconque des revendications 1 à 21, ledit article étant capable de former un film continu pelable après dissolution du support et comportant un ou plusieurs agents filmogènes.

23. Article selon l'une quelconque des revendications 1 à 20, **caractérisé par le fait qu'**il contient au moins un organopolysiloxane hydrophile élastomérique solide.

24. Article selon l'une quelconque des revendications précédentes, le support formant un coussinet, un masque ou un patch, une charlotte, un doigt de gant ou un gant, une nappe à découper ou une lingette, un disque, un oval ou un rectangle.

25. Article selon l'une quelconque des revendications 1 à 24, l'article étant sec au toucher avant l'utilisation.

26. Article selon l'une quelconque des revendications 1 à 25, **caractérisé par le fait qu'**il comporte une composition de maquillage représentant entre 10 et 1000 % en poids, par rapport au poids du support.

27. Ensemble comportant :
- un emballage,
- au moins un article tel que défini dans l'une quelconque des revendications 1 à 26.

28. Ensemble selon la revendication précédente, **caractérisé par le fait que** l'article comporte une composition de maquillage colorée et **par le fait que** l'emballage comporte un témoin coloré représentatif de la couleur de la composition de maquillage.

## Patentansprüche

1. Kosmetischer Artikel, der aufweist:
- einen Träger in der Form von zumindest einem Vlies von einem in Wasser bei einer Temperatur von weniger als 20 °C löslichen Material, und welches eine Dichte von weniger als oder gleich 0,1 g/m³ vorzuweisen hat, und
- zumindest eine Zusammensetzung von einer Makeup- bzw. einer Schminkzusammensetzung, die durch den Träger getragen wird, wobei der Artikel ein Mittel zum Färben aufweist, das ausgewählt ist aus: den mineralischen Pigmenten, den organischen Pigmenten oder Lacken, den perlmuttartig glänzenden Pigmenten, den Zusammensetzungen von Pigmenten, den in Wasser löslichen und in Fett löslichen Färbemitteln, den mineralischen Pigmenten, die ausgewählt sind aus: dem Titandioxid, das an der Oberlfäche behandelt worden ist, den Oxiden des Zirkoniums und des Zeriums, wie auch den Oxiden des Eisens oder des Chromes, dem Magnesiumviolett, dem Ultramarin, dem Chromhydrat und dem Eisenblau.

2. Artikel nach dem Anspruch 1, **dadurch gekennzeichnet, dass** dieser flexibel ist.

3. Artikel nach dem Patentanspruch 1, **dadurch gekennzeichnet, dass** dieser nicht klebend ist.

4. Artikel nach einem der Ansprüche 1 bis 3, **dadurch gekennzeichnet, dass** der Träger vollständig in Wasser löslich ist.

5. Artikel nach irgendeinem der Ansprüche 1 bis 4, in welchem der Träger Fasern aufweist, die in Wasser löslich sind.

6. Artikel nach dem Anspruch 5, in welchem der Träger im Wesentlichen aus den besagten löslichen Fasern gebildet wird.

7. Artikel nach irgendeinem der Ansprüche 1 bis 6, in welchem der Träger zumindest eine Schicht bzw. Lage, die nicht gewebt ist, aufweist, die im Wesentlichen aus Fasern gebildet ist, die in Wasser löslich sind.

8. Artikel dem voranstehenden Anspruch, **dadurch gekennzeichnet, dass** der Träger zumindest zwei nicht gewebte Schichten bzw. Lagen aufweist, die jeweils im Wesentlichen aus in Wasser löslichen Fasern gebildet sind.

9. Artikel nach dem voranstehenden Anspruch, **dadurch gekennzeichnet, dass** die zwei Schichten bzw. Lagen durch zwei Blätter gebildet werden, die an ihrer Peripherie zusammengesetzt bzw. montiert sind.

10. Artikel nach dem voranstehenden Anspruch, **dadurch gekennzeichnet, dass** die Blätter thermisch verschweißt sind.

11. Artikel nach Anspruch 2, **dadurch gekennzeichnet, dass** die Makeup- bzw. Schminkzusammensetzung zumindest teilweise in dem Inneren des Trägers dispergiert ist.

12. Artikel nach dem Anspruch 8, **dadurch gekennzeichnet, dass** die Makeup- bzw. Schminkzusammensetzung zumindest teilweise zwischen den Schichten bzw. Lagen, besser genau zwischen den Schichten bzw. Lagen untergebracht ist.

13. Artikel nach irgendeinem der voranstehenden Ansprüche, der eine im Wesentlichen abgeflachte Form vorzuweisen hat.

14. Artikel nach irgendeinem der Ansprüche 1 bis 12, welcher einen Block ausbildet.

15. Artikel nach dem Anspruch 1, **dadurch gekennzeichnet, dass** dieser zumindest eine Schicht bzw. Lage aufweist, die im Wesentlichen aus in Wasser löslichen Fasern und zumindest einer Schicht bzw. Lage eines Substrates gebildet ist, das in Wasser unlöslich ist.

16. Artikel nach dem Anspruch 5 oder 6, in welchem die Fasern Polyvinylalkohol aufweisen.

17. Artikel nach dem Anspruch 16, in welchem die Fasern einen transversal ebenen Querschnitt vorzuweisen haben, der genau kreisförmig ist.

18. Artikel nach irgendeinem der voranstehenden Ansprüche, **dadurch gekennzeichnet, dass** die Makeup- bzw. Schminkzusammensetzung eine pulvrige Zusammensetzung ist.

19. Artikel nach irgendeinem der Ansprüche 1 bis 17, **dadurch gekennzeichnet, dass** die Makeup- bzw. Schminkzusammensetzung eine pastöse Zusammensetzung ist.

20. Artikel nach irgendeinem der Ansprüche 1 bis 17, **dadurch gekennzeichnet, dass** die Schmink- bzw. Makeup-Zusammensetzung eine schäumende Zusammensetzung ist.

21. Artikel nach irgendeinem der Ansprüche 1 bis 20, **dadurch gekennzeichnet, dass** dieser eine Makeup- bzw. Schminkzusammensetzung für die Haut, die Schleimhäute oder die Hautanhangsgebilde bzw. Phanere aufweist.

22. Artikel nach irgendeinem der Ansprüche 1 bis 21, wobei der Artikel dazu in der Lage ist, einen durchgehenden bzw. fortgesetzten Film auszubilden, der von dem Träger nach der Auflösung abziehbar ist und ein oder mehrere Filmbildungsmittel aufweist.

23. Artikel nach irgendeinem der Ansprüche 1 bis 22, **dadurch gekennzeichnet, dass** dieser zumindest einen organopolysiloxanhydrophilelastomeren Festkörper enthält.

24. Artikel nach irgendeinem der voranstehenden Ansprüche, wobei der Träger ein Lager, eine Maske oder einen Flecken, eine Charlotte, ein Schutzrohr oder einen Handschuh, eine Lage zum Abtrennen oder einen Wischer, eine Scheibe, ein Oval oder ein Rechteck ausbildet.

25. Artikel nach irgendeinem der Ansprüche 1 bis 24, wobei der Artikel vor der Benutzung bei Berührung trocken ist.

26. Artikel nach irgendeinem der Ansprüche 1 bis 25, **dadurch gekennzeichnet, dass** dieser eine Makeup- bzw. Schminkzusammensetzung aufweist, die zwischen 10 und 1000 Gew.-% im Vergleich zu dem Gewicht des Trägers aufweist.

27. Anordnung, die aufweist:
- eine Verpackung bzw. einen Behälter,
- zumindest einen Artikel, wie dieser in irgendeinem der voranstehenden Ansprüche 1 bis 26 definiert ist.

28. Anordnung nach dem voranstehenden Anspruch, **dadurch gekennzeichnet, dass** der Artikel eine färbende Makeup- bzw. Schminkzusammensetzung aufweist, und dadurch, dass der Behälter bzw. die Verpackung eine Farbanzeige aufweist, die für die Farbe der Makeup- bzw. Schminkzusammensetzung repräsentativ ist.

## Claims

1. A cosmetic article including:
- a support in the form of at least one layer of a water-soluble material at a temperature of less than 20°C, and having a specific gravity of less than or equal to 0.1 g/cm³, and
- at least one make-up composition borne by the support, the article including a coloring agent selected from among: mineral pigments, organic pigments or lacquers, mother-of-pearl pigments, composite pigments, oil-soluble and water-soluble coloring agents, the mineral pigments being selected from among: surface-treated titanium dioxide, zirconium or cerium oxides, as well as iron or chromium oxides, manganese violet, ultramarine blue, chromium hydrate and ferric blue.

2. The article according to claim 1, **characterized by** the fact that it is flexible.

3. The article according to claim 1, **characterized by** the fact that it is non-adhesive.

4. The article according to any of claims 1 to 3, **characterized by** the fact that the support is entirely soluble in water.

5. The article according to any of claims 1 to 4, wherein the support includes water-soluble fibers.

6. The article according to claim 5, wherein the support essentially consists of said water-soluble fibers.

7. The article according to any of claims 1 to 6, wherein the support includes at least one layer of a non-woven fabric essentially consisting of water-soluble fibers.

8. The article according to the preceding claim, **characterized by** the fact that the support includes at least two layers of non-woven fabric each essentially consisting of water-soluble fibers.

9. The article according to the preceding claim, **characterized by** the fact that both layers are formed with two sheets assembled at their periphery.

10. The article according to the preceding claim, **characterized by** the fact that the sheets are heat-welded.

11. The article according to claim 2, **characterized by** the fact that the make-up composition is at least partly dispersed within the support.

12. The article according to claim 8, **characterized by** the fact that the make-up composition is at least partly positioned between the layers, better, positioned substantially entirely between the layers.

13. The article according to any of the preceding claims, having a general flattened shape.

14. The article according to any of claims 1 to 12, forming a block.

15. The article according to claim 1, **characterized by** the fact that it includes at least one layer essentially consisting of water-soluble fibers and at least one layer of a substrate insoluble in water.

16. The article according to claim 5 or 6, wherein the fibers include polyvinyl alcohol.

17. The article according to claim 16, wherein the fibers have a substantially circular solid cross-section.

18. The article going to any of the preceding claims, **characterized by** the fact that the make-up composition is a powdery composition.

19. The article according to any of claims 1 to 17, **characterized by** the fact that the make-up composition is a pasty composition.

20. The article according to any of claims 1 to 17, **characterized in that** the make-up composition is a foaming composition.

21. The article according to any of claims 1 to 20, **characterized by** the fact that includes a make-up composition for the skin, the mucosae, or the appendages.

22. The article according to any of claims 1 to 21, said article being capable of forming a peelable continuous film after dissolution of the support and including one or several film-forming agents.

23. The article according to any of claims 1 to 20, **characterized by** the fact that it contains at least the one solid elastomeric hydrophilic organopolysiloxane.

24. The article according to any of the preceding claims, the support forming a pad, a mask or a patch, a headwear, a glove finger or a glove, a cloth to be cutout or a wipe, a disc, an oval or a rectangle.

25. The article according to any of claims 1 to 24, the article being dry when touched before use.

26. The article according to any of claims 1 to 25, **characterized by** the fact that it includes a make-up composition representing between 10 and 1,000% by weight based on the weight of the support.

27. An assembly including:
- a packaging,
- at least one article as defined in any of claims 1 to 26.

28. The assembly according to the preceding claim, **characterized by** the fact that the article includes a colored make-up composition and by the fact that the package includes a colored indicator representative of the color of the make-up composition.
